# EUROPEAN PATENT SPECIFICATION

(11) **EP 3 016 931 B1**
(45) Date of publication and mention of the grant of the patent: **24.02.2021**
(21) Application number: 14738726.0
(22) Date of filing: 19.06.2014
(51) Int. Cl.: C07C 233/18, C07C 41/18, C07C 41/22, C07C 43/225, C07C 217/60, C07C 213/02, C07C 231/02

(54) **A NOVEL PROCESS FOR THE PREPARATION OF TETRALIN AND NAPHTHALENE DERIVATIVES**
NEUARTIGES VERFAHREN ZUR HERSTELLUNG VON TETRALIN- UND NAPHTHALINDERIVATEN
NOUVEAU PROCÉDÉ POUR LA PRÉPARATION DE DÉRIVÉS DE TÉTRALINE ET DE NAPHTALÈNE

(30) Priority: 04.07.2013 WO PCT/EP2013/001959
(43) Date of publication of application: 11.05.2016
(73) Proprietor: Pharmathen S.A., 15351 Pallini Attikis (GR)
(72) Inventor: KOFTIS, V. Theocharis, GR-57001 Thessaloniki (GR); NEOKOSMIDIS, Efstratios, GR-57001 Thessaloniki (GR); STATHAKIS, Christos, GR-57001 Thessaloniki (GR); ANDREOU, Thanos, GR-57001 Thessaloniki (GR); RAPTIS, Christos, GR-57001 Thessaloniki (GR); VARVOGLI, Anastasia-Aikaterini, GR-57001 Thessaloniki (GR)
(86) International application number: PCT/EP2014/001672
(87) International publication number: WO 2015/000555

(56) References cited:
- EP-A1- 1 564 202
- EP-A1- 2 562 151
- WO-A2-2012/046253
- MOHAMED ETTAOUSSI ET AL: "Design, synthesis and pharmacological evaluation of new series of naphthalenic analogues as melatoninergic (MT/MT) and serotoninergic 5-HTdual ligands (I)", EUROPEAN JOURNAL OF MEDICINAL CHEMISTRY, EDITIONS SCIENTIFIQUE ELSEVIER, PARIS, FR, vol. 49, 12 January 2012 (2012-01-12), pages 310-323, XP028458565, ISSN: 0223-5234, DOI: 10.1016/J.EJMECH.2012.01.027 [retrieved on 2012-01-20]

## Description

### TECHNICAL FIELD OF THE INVENTION

The present invention relates to a novel process for the preparation of tetralin and naphthalene derivatives. Such compounds are considered to be interesting either as useful building blocks or due to their biological activity.

### BACKGROUND OF THE INVENTION

Compounds comprising of bicyclic cores such as the naphthalenic or the tetralinic core are of particular interest, either because they possess biological activity, or because they are valuable intermediates for the synthesis of others. Accordingly, there is a continuous interest towards the development of new synthetic methods for such compounds.

In EP 1831159 tetralin derivatives are prepared, for use in the treatment of CNS diseases and gastrointestinal tract disorders, starting from the fluoro-tetralone shown in the following scheme.

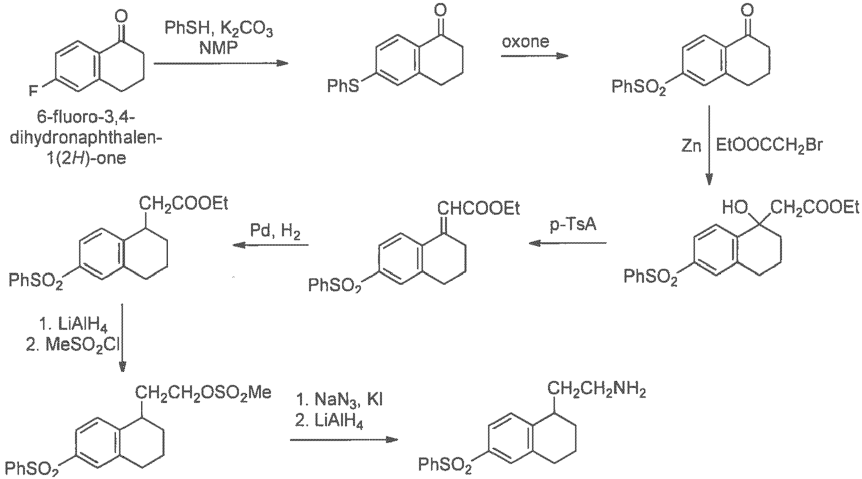

According to this process, it takes five steps to obtain the amine from the respective phenylsulphonyl intermediate and, in addition, it uses LiAlH₄ and NaN3, which are known to be difficult to handle in industrial scale.

In EP 0728738 tetralin (and indan) derivatives are prepared according to the following scheme.

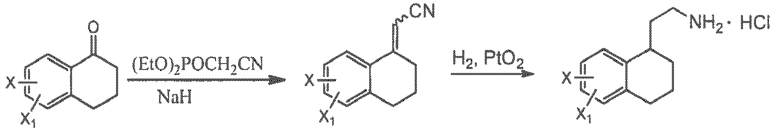

This synthetic route requires the use of metal catalyst for the hydrogenation step.

EP 1564202 prepares Agomelatine, a compound used for the treatment of major depressive disorder (EP0447285B1), from 7-methoxy-tetralone: 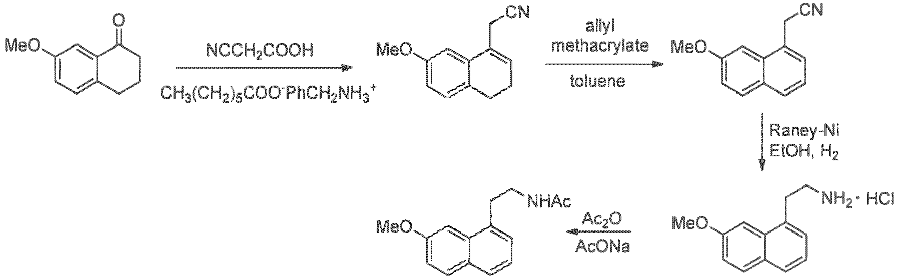 This process is quite short, but employs the use of benzylamine and heptanoic acid, substances which exhibit toxicity. 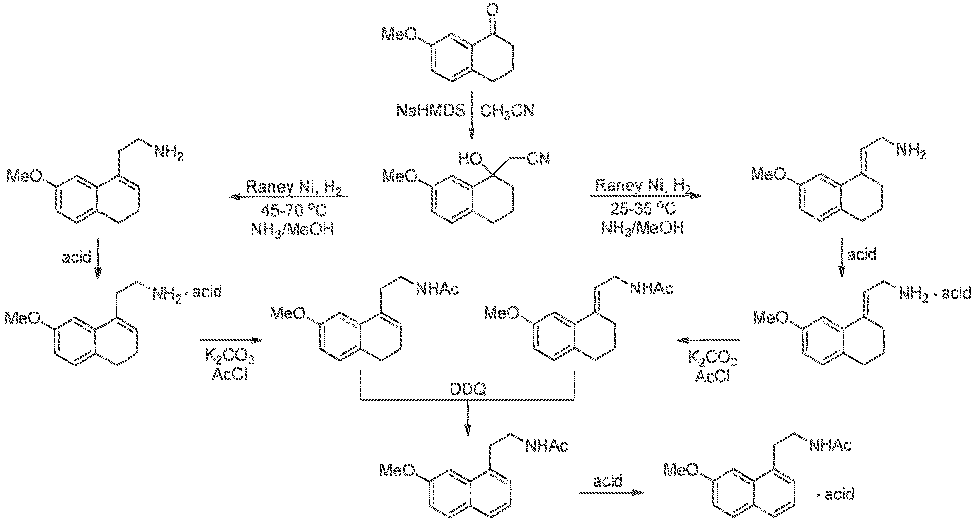 WO2012046253A2 prepares Agomelatine according to the above scheme. The process needs very low temperatures for the performance of the first step (below -70 °C), conditions difficult to maintain in manufacturing processes. It is also considered that the second step, which includes reduction of the CN group and elimination towards a formation of a double bond, is a reaction with conflicting reports found in the prior art.

In EP2562151A1 Agomelatine is prepared through a chloroethylnaphthalene derivative prepared according to following scheme. The process continues with two possible routes: i) formation of the respective phthalimide and hydrolysis, ii) formation of the respective nitro-derivative and subsequent catalytic hydrogenation, iii) formation of the respective N,N-diformylaminoethyl derivative, followed by hydrolysis. The ethylamine intermediate obtained is then acetylated, under known conditions, to Agomelatine. 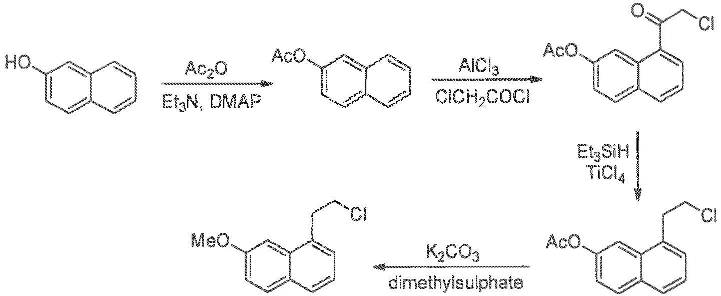 The process is also very long and includes the use of dimethylsulphate, which is a known genotoxic substance.

In view of the above disadvantages there is still a need for a synthetic process towards such naphthalene and tetraline derivatives which will use safer reagents, will be short and at the same time suitable for industrialization.

### SUMMARY OF THE INVENTION

The present invention discloses a novel process for the preparation of compounds of formula IVa 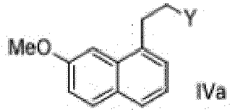 in which
Y is selected from hydroxyl or protected hydroxyl group, halogen, amino or alkylamino group, amido or substituted amido group, or phthalimide group, wherein:
protected hydroxyl means a hydroxyl group suitably protected with a protecting group;
alkylamino means an amino group substituted with one or two of the same or different alkyl groups;
amido means -NHCOX wherein X is selected from an alkyl, alkoxy,
alkoxyalkyl or alkylamino group;
substituted amido means -N(Z)COX, wherein X is defined as above and Z is alkyl;
comprising the following steps:
   a) allylic rearrangement of a compound of formula la, 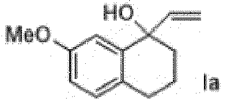 to form compound of formula IIa 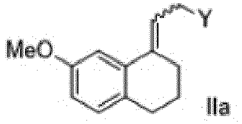 which may be optionally isolated;
   b) isomerization of a compound of formula IIa to form a compound of formula IIIa 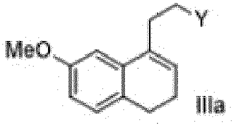 wherein Y is defined as above, which may be optionally isolated;
   c) conversion of a compound of formula IIIa to a compound of formula IVa, which may be optionally isolated as a free compound or a salt 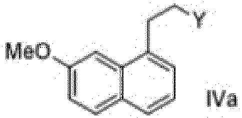 wherein Y is defined as above.

According to another object of the present invention, the process described above may further comprise step d), wherein Y is converted to an amido group, preferably NHCOCH₃, when Y is other than an amido group.

A particular object of the present invention is a previously undisclosed allylic rearrangement of a compound of Formula Ia that enables the preparation of a compound of Formula IIa.

A further object of the present invention is the preparation of a compound of Formula IIIa from a compound of Formula Ia or IIa comprising an allylic rearrangement step.

According to a further object of the invention, steps a) and b) of the process described above may be performed without isolation of any intermediate compound.

According to another object of the invention, steps a), b) and c) of the process described above may be performed without isolation of any intermediate compound.

A further object of the present invention is a novel process for the preparation of a compound of Formula IVa from a compound of Formula Ia. 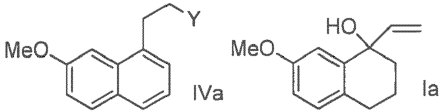 wherein A and Y are defined as above

In particular, the present invention discloses the preparation of a compound of Formula IVa from a compound of Formula Ia through a novel process comprising an allylic rearrangement step.

The novel process for the transformation of a compound of Formula Ia to a compound of Formula IVa comprises the following steps:
a) allylic rearrangement of compound of formula Ia, providing a compound of formula IIa, wherein Y is defined as above 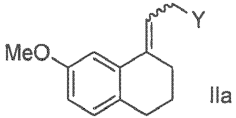
b) isomerization, providing a compound of formula IIIa, wherein Y is defined as above 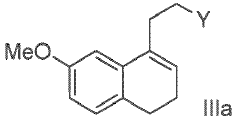
c) conversion to a compound of formula IVa, wherein Y is defined as above.

In a preferred embodiment of the present invention, Y is a halogen atom, more preferably a chloro atom. According to a further object of the present invention, the process described above may further comprise step d), wherein Y is converted to an amido group, preferably NHCOCH₃, when Y is other than an amido group.

In a preferred embodiment, step d) Y is preferably a halogen atom, even more preferably chloro atom.

According to a further object of the invention, steps a) and b) of the process described above may be performed without isolation of any intermediate compound.

According to another object of the invention, steps a), b) and c) of the process described above may be performed without isolation of any intermediate compound.

A particular object of the present invention is a previously undisclosed allylic rearrangement of a compound of Formula Ia that enables the preparation of a compound of Formula IIa.

A further object of the present invention is the preparation of a compound of Formula IIIa from a compound of Formula Ia or IIa through an allylic rearrangement step.

In a preferred embodiment of the present invention, the preparation of compounds IIa and IIIa is provided though the processes described above, wherein Y is preferably a halogen atom, most preferably a chloro atom.

A further object of the present invention is a novel process for the preparation of agomelatine (compound of Formula VI) or a salt thereof, from a compound of Formula Ia.

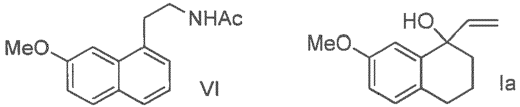

In particular, the present invention discloses the preparation of agomelatine of formula VI or a salt thereof from a compound of Formula Ia through a novel process comprising an allylic rearrangement step.

In a further object of the present invention, the process described above may further comprise converting compound of formula IVa to a compound of formula V, which is then converted to compound of formula VI.

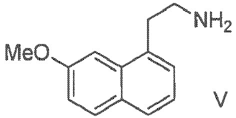

In another object of the present invention, the process described above may further comprise converting compound of formula IVa to a compound of formula VII, as defined below, further converted to compound of formula V and then to compound of formula VI.

### General Definitions

The term "aromatic ring" means a C₆₋₁₈ aromatic group, formed by one or more rings, which may be substituted with one or more substituents, or unsubstituted. Preferably, the aromatic ring is a C₆₋₁₀ ring system. Typical examples include phenyl, naphthyl and anthracenyl.

The term "heterocyclic ring" means an aromatic ring, defined as above, which contains one or more heteroatoms. Suitable heteroatoms will be apparent to those skilled in the art and include, for example, nitrogen, oxygen, sulphur, phosphorus and silicon. Preferable heteroatoms are nitrogen, oxygen and sulphur.

The term "alkyl" means a monovalent straight or branched chain group of the formula CₘH₂ₘ₊₁ or a cyclic group of the formula CₘH₂ₘ₋₁, with m being the number of carbon atoms. Preferable alkyl groups are C₁₋₂₀ alkyl groups, more preferably C₁₋₁₀ alkyl groups, more preferably C₁₋₈ alkyl groups. Particularly preferred alkyl groups include, for example, methyl, ethyl, *n*-propyl, *iso*-propyl, *n*-butyl, *sec*-butyl, *iso*-butyl, *n*-pentyl, *n*-hexyl, *n*-heptyl, *n*-octyl.

The term "haloalkyl" refers to alkyl groups substituted with one or more halogen atoms in one or more positions of the alkyl chain.

The term "alkenyl" means monovalent straight or branched chain moieties containing one or more carbon-carbon double bonds and at least 2 carbon atoms. These moieties conform to the formula CₘH₍₂ₘ₋₁₎, with m being the number of carbon atoms present. Preferable alkenyl groups are C₂₋₂₀ alkenyl groups, more preferably still a C₂₋₁₀ alkenyl group, more preferably still a C₂₋₁₀, more preferably still a C₂₋₈ alkenyl group.

The term "alkynyl" means monovalent straight or branched chain moieties containing one or more carbon-carbon triple bonds and at least 2 carbon atoms. These moieties conform to the formula CₘH₍₂ₘ₋₃₎, with m being the number of carbon atoms present. Preferable alkynyl groups are C₂₋₂₀ alkynyl groups, more preferably still a C₂₋₁₀ alkynyl group, more preferably still a C₂₋₁₀ alkynyl, more preferably still a C₂₋₈ alkynyl group.

The term "aryl" refers to an aromatic ring system, as defined above.

The term "substituted hydroxy" means -O-alkyl, -O-aryl, -O-C(O)-alkyl, -O-C(O)-aryl, -O-SO₂-O-alkyl, -O-SO₂-O-haloalkyl, -O-SO-₂-O-aryl. Preferable substituted hydroxyl groups are -O-C₁₋₈ alkyl, -O-C₆₋₁₀ aryl, -O-C(O)-C₁₋₈-alkyl, -O-C(O)-C₆₋₁₀-aryl, -O-SO₂-O-C₁₋₈ alkyl, -O-SO₂-O-C₁₋₈ alkyl substituted with one or more halogen atoms, -O-SO-₂-O-C₆₋₁₀ aryl. More preferable substituted hydroxyl groups are -O-methyl, -O-ethyl, O-*n*-propyl, O-*i*-propyl, -O-*n*-butyl, -O-*i*-butyl, -O-*sec-*butyl, -O-phenyl, -O-p-tolyl, -O-1-naphthyl, - O-2-naphthyl, are -O-C(O)-methyl, -O-C(O)-ethyl, -O-C(O)-*n*-propyl, -O-C(O)-*i*-propyl, -O-C(O)-phenyl, -O-SO₂-methyl, - O-SO₂-trifluoromethyl, -O-SO₂-p-tolyl. More preferable substituted hydroxyl groups are -O-methyl, -O-ethyl, O-*n*-propyl, O-*i*-propyl, -O-phenyl, -O-p-tolyl, -O-SO₂-methyl, -O-SO₂- trifluoromethyl, -O-SO₂-p-tolyl. More preferable substituted hydroxyl groups are -O-methyl, -O-ethyl, O-*n*-propyl, O-*i*-propyl, -O-SO₂-methyl, -O-SO₂-trifluoromethyl, -O-SO₂-p-tolyl.

The term "aminoalkyl" means an amino group substituted with one or two of the same or different alkyl groups. Preferred aminoalkyl groups are -N (C₁₋₂₀-alkyl)₂. More preferred aminoalkyl groups are -N(C₁₋₈-alkyl)₂.

The term "alkoxycarbonyl" means alkyl-O-C(O)- groups. Preferred alkoxycarbonyl groups are (C₁₋₂₀ alkyl)-O-C(O)- groups, more preferably (C₁₋₈-alkyl)-O-C(O)- groups.

The term "protected hydroxyl" refers to hydroxyl groups suitably protected with protecting groups. Such groups are known in the art and are exemplified such as in Greene's Protective Groups on Organic Synthesis 4th Edition, John Wiley & Son, Peter G. M. Wuts, Theodora W. Greene, Print ISBN: 9780471697541. Preferred protecting groups are trityl, benzyl, naphthyl, methoxybenzyl, p-nitrobenzyl, benzoyl, a substituted benzoyl, trimethylsilyl, triethylsilyl, isopropyldimethylsilyl, tert-butyldimethylsilyl, tert-butyldiphenylsilyl, thexyldimethylsilyl, allyl, methoxymethyl, (2-methoxyethoxy)methyl, tetrahydropyranyl.

The term substituted amino means an amino group substituted with one or two alkyl, alkoxy, alkoxyalkyl, alkylocarbonyl groups, wherein alkoxy is alkyl-O-, alkoxyalkyl is alkyl-O-alkyl-, aminoalkyl is defined as above and wherein each alkyl is preferably as defined above.

The term "amido" means -NHCOX, wherein X is alkyl, alkoxy, alkoxyalkyl, alkylocarbonyl or aminoalkyl, as defined above.

The term "substituted amido" means -N(Z)COX, wherein X is defined as above and Z is alkyl.

The term phthalimido refers to the radical

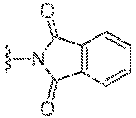

Where a term defined above is described as substituted, examples of suitable substituents may include one or more of halogen, alkyl, haloalkyl, cycloalkyl, alkenyl, alkynyl, aryl, halo, hydroxyl, substituted hydroxy, amino, substituted amino, nitro, cyano, formyl, alkyl carbonyl, alkoxycarbonyl, carboxyl.

### Salts

It will be appreciated by the person skilled in the art that, whenever possible, compounds of the formulae IIa, IIIa, IVa, V and VI may also exist in the form of addition salts. Acceptable salts of the compounds prepared herein include suitable acid addition or base salts thereof. A review of suitable pharmaceutical salts may be found in Berge et al., J. Pharm. Sci., 66, 1, 19 (1977). Salts are formed, for example, with strong acids such as mineral acids, e. g. sulphuric acid, phosphoric acid or hydrohalic acids; with strong organic carboxylic acids, such as alkanecarboxylic acids of 1 to 4 carbon atoms which are unsubstituted or substituted (e. g., by halogen), such as acetic acid and trifluoroacetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁₋₄)-alkyl- or aryl-sulfonic acids which are substituted or unsubstituted (for example, by a halogen) such as methane- or p-toluene sulfonic acid. Salts which are not pharmaceutically acceptable may still fall within the scope of the invention.

### Hydrates and solvates

It will further be appreciated by the person skilled in the art that the compounds prepared in the present invention may be isolated as solvates, hydrates or anhydrous forms. Therefore, the term "compound" can be interpreted as referring to any of the above forms.

### Stereoisomers

It will be acknowledged, by the person skilled in the art that compounds of formula I can occur in different diastereomeric forms or mixtures thereof. Although it is not important for the rearrangement step, any diastereomer or mixture of the diastereomers is within the scope of the invention.

It will further be acknowledged, by the person skilled in the art, that the isomerization of compounds of formula IIa of any composition, with respect to their geometrical isomers, is leading to the corresponding endocyclic compounds of formula IIIa.

### DETAILED DESCRIPTION OF THE INVENTION

The invention provides a process for the preparation of compounds of formula IVa 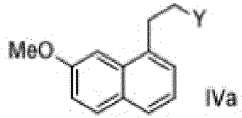 in which
Y is selected from hydroxyl or protected hydroxyl group, halogen, amino or alkylamino group, amido or substituted amido group, or phthalimide group, wherein:
protected hydroxyl means a hydroxyl group suitably protected with a protecting group;
alkylamino means an amino group substituted with one or two of the same or different alkyl groups;
amido means -NHCOX wherein X is selected from an alkyl, alkoxy,
alkoxyalkyl or alkylamino group;
substituted amido means -N(Z)COX, wherein X is defined as above and Z is alkyl ;
comprising the following steps:
   a) allylic rearrangement of a compound of formula la, 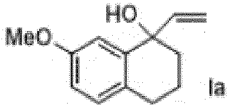 to form compound of formula IIa 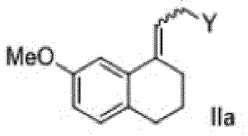 which may be optionally isolated;
   b) isomerization of a compound of formula IIa to form a compound of formula IIIa 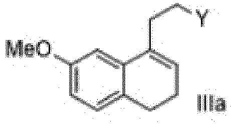 wherein Y is defined as above, which may be optionally isolated;
   c) conversion of a compound of formula IIIa to a compound of formula IVa, which may be optionally isolated as a free compound or a salt 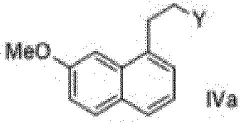 wherein Y is defined as above.

In another embodiment of the present invention, the above described process may further comprise step d), wherein Y is converted to an amido group, preferably NHCOCH₃, when Y is other than an amido group.

In step a), the rearrangement is performed under conditions which can either induce transformation of the OH group of compound of formula Ia to a good leaving group, or under conditions comprising halogenating reagents, Brownsted-Lowry acids, or Lewis acids.

The reaction can run in typical organic solvents such as alcohols, for example methanol, ethanol or similar alcohols; ethers, for example diethyl ether; tetrahydrofuran (THF) or similar, or halogenated solvents, for example dichloromethane, chloroform or similar. The temperature of the rearrangement can vary from about -30 °C to the boiling point of the selected solvent. Compound of Formula IIa may be optionally isolated.

In step b), the isomerization takes place in commonly used organic solvents that can efficiently solvate compound of Formula II. Compound of Formula III may be optionally isolated.

In step c) the preparation of compound of Formula IVa can be achieved by known in the art methods for the dehydrogenation of partially hydrogenated aromatic compounds. For example the dehydrogenation of compound of Formula IIIa can be accomplished using S₈, 2,3-dichloro-5,6-dicyano-1,4-benzoquinone (DDQ), p-chloranil or transition metal catalysis in the presence of a hydrogen acceptor.

In step d) a substitution reaction and an acylation reaction are involved. The substitution step may be achieved with a nitrogen source. Non limiting examples are ammonia, amines of general type RNH₂, R₂NH, dialkyl phosphoramidite compounds of general type H₂NP(O)(OR)₂, phthalimide derivatives (e. g. salts thereof, such as potassium phthalimide), azide derivatives (e. g. salts thereof, such as sodium azide) and sulfonamides of general type RSO₃NH₂, wherein is R an alkyl or aryl group, defined as above. The acylation step may be performed with an alkyl halide or an acyl anhydride in the presence of a base.

In another embodiment of the invention, we unexpectedly discovered that step c) may also be performed with a catalytic amount of DDQ. This feature is an important improvement over prior art methods, due to the fact that DDQ is considered genotoxic and moreover leads to laborious workup procedures. Therefore, the significant reduction of the amount used in an industrial process is a major advantage of the present invention. DDQ, after performing the aromatization reaction may be regenerated by using a suitable catalytic cycle involving one or more co-oxidants. Non limiting examples of co-oxidants are PbO₂, HClO₄/H₂SO₄, HIO₄, MnO₂, Mn(OAc)3, tert-butylnitrite (TBN)/O₂, NaNO₂/O₂, FeCl₃, Pd(OAc)₃, HNO₃.

In a preferred embodiment of the invention, the substitution step is performed with aqueous ammonia.

In an embodiment of the process described above, the present invention discloses the preparation of a compound of Formula IVa from a compound of Formula Ia.

In still another embodiment of the present invention, step a) may be performed under acidic conditions involving Brownsted-Lowry acids. Non limiting examples are hydrohalic acids, sulfuric acid, acetic acid or similar. The step may also be performed with Lewis acids, such as metal halides, semimetal halides and non metal halides, for example TiHal₄, LiHal, or halogenating reagents such as thionyl halide, phosphorus halides, oxalyl halide. The step may also be performed with compounds that can transform the hydroxyl group into a good leaving group, for example acetyl chloride, p-tosyl halide, trimethylsilyl halide. The solvent of the reaction can be selected among commonly used organic solvents such as ethers, for example diethyl ether, THF or similar or halogenated solvents, for example dichloromethane, chloroform or similar. The temperature of the rearrangement can vary from about 0 °C to the boiling point of the selected solvent. Compound of Formula IIa may be optionally isolated.

The solvent used in the reaction of step b can be selected among typical organic solvents, preferably but not restricted to halogenated solvents such as dichloromethane, chloroform or 1,2-dichloroethane or ethers such as diethyl ether, THF or similar. The isomerization occurs at temperatures between about 0 °C and about 100 °C. Compound of Formula IIIa may be optionally isolated.

The dehydrogenation of compound of Formula IIIa takes place by known in the art methods for the dehydrogenation of partially hydrogenated aromatic compounds. In an embodiment of the present invention, the transformation could be efficiently accomplished using DDQ or p-chloranil in commonly used organic solvents and at ambient temperature. The said oxidation is completed in short reaction times, usually in a course of a few hours, but may require longer reaction time depending on the compound.

In another embodiment of the invention the dehydrogenation could be achieved by the action of catalytic amounts of Pd in the presence of a hydrogen acceptor. As hydrogen acceptors unsaturated compounds such as 1-dodecene, allyl methacrylate or compounds bearing reducible functional groups such as nitroalkanes or nitroarenes can be utilized. The reaction typically requires heating at temperatures above 100 °C for a course of about 12 h or longer.

A further object of the present invention is a novel method for the preparation of Agomelatine (compound of Formula VI) or a salt thereof, from a compound of Formula Ia 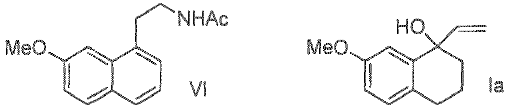 comprising the following steps:
a) conversion of compound of Formula Ia to a compound of Formula IIa where Y is chloro through an allylic rearrangement. 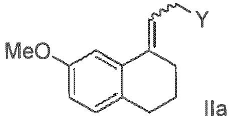
b) isomerization of compound of Formula IIa to a compound of Formula IIIa where Y is chloro. 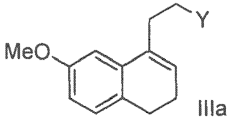
c) conversion to a compound of Formula IVa
d) conversion of compound of Formula IVa, where Y is choro, to a compound of Formula VI or any salt thereof.

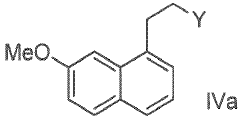

In a preferred embodiment of the invention, compound of Formula Ia is treated with concentrated aqueous HCl. The amount of the acid required is typically between 2 and 3 times the amount by molar of the starting material. The rearrangement takes place in common organic solvents such as THF, diethyl ether, tert-butyl methyl ether, preferably in THF. The temperature of the reaction ranges from about 0 °C to about 40 °C, typically being between 20-30 °C. Compound of formula Ia is fully consumed usually within 0.5 h to 2 h. The reaction mixture is worked up using known methods in the art.

In an embodiment of the invention a solution of compound of formula IIa in common organic solvents, typically selected among halogenated ones such as dichloromethane, chloroform, 1,2-dichloroethane or similar is stirred at temperature ranging between 20 and 60 °C, preferably between 20-30 °C. The isomerization process usually requires about 12 h to about 24 h to be completed. The reaction mixture is worked up using known methods in the art.

In a further embodiment of the invention, the aromatization of compound of Formula IIIa could be accomplished using DDQ, in 1 to 2 fold molar excess, in any low-polar organic solvent, preferably toluene or dichloromethane (DCM). The transformation is completed in short reaction times, typically 1-2 hours, at ambient temperature. Compound of Formula IIIa can also be dehydrogenated using various palladium sources such as Pd metal or Pd(OH)₂ on activated carbon in the presence of a hydrogen acceptor. In a preferred embodiment, allyl methacrylate is utilized as the hydrogen acceptor in 3 fold molar excess compared to compound IIIa. The reaction is performed in common organic solvents such as toluene, THF, ethyl acetate, MeOH, with the optimum choice being toluene. Typically the reaction time varies from about 12 h to about 48 h at a temperature range from 100 °C to 130 °C and the product can be isolated by a simple filtration through a celite pad.

In another embodiment of the present invention, the aromatization step can be performed with a catalytic amount of DDQ using as a co-oxidant NaNO₂ in the presence of oxygen gas. The amount of DDQ required for the completion of the transformation can vary from 0.1 to 0.3 folds the molar amount of the substrate. In a preferred embodiment, this amount can be as low as 0.1 folds the molar amount of the substrate. In accordance, the amount of NaNO₂ can be equal or greater than the amount of DDQ used. In a preferred embodiment the amount of co-oxidant NaNO₂ is the same as the molar amount of DDQ, in which case the presence of oxygen gas is required. The reaction means can be selected among various medium to high polar aprotic solvents such as ethyl acetate, acetone, acetonitrile. Preferably the aromatization step is performed in acetonitrile. The temperature of the reaction can vary from 30 to 110°C, preferably between 60 and 100°C. More specifically the aromatization takes place by heating between 80-95°C.

In an aspect of the invention compound of formula IVa, wherein Y is chloro, is converted to agomelatine.

In a further aspect of the invention, compound IVa (Y is chloro) is converted to the hydrochloride salt of compound V, using various nitrogen sources.

In a preferred embodiment, the transformation can be achieved directly through a substitution reaction using aqueous ammonia as the nucleophile. The substitution is achieved by heating a biphasic system at a temperature range between 80 and 120 °C and typically is completed within a course of a few hours.

In another embodiment the said transformation can be succeeded through a two-step process comprising conversion of compound of formula IVa, where Y is chloro, to a compound of formula IVa, where Y is phthalimide radical and subsequent hydrolysis to compound of Formula V.

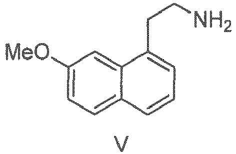

Compound IVa (Y is phthalimide radical) is prepared using potassium phthalimide as the nitrogen source under heating at about 100 °C in polar solvents such as dimethylformamide. Conversion of phthalimide IVa to compound V is accomplished by hydrazinolysis in protic solvents such as methanol or ethanol.

In a further embodiment of the invention, acetylation of compound of Formula V or a salt thereof forms compound of Formula VI (Agomelatine).

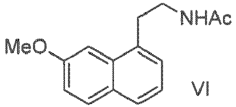

In a preferred embodiment, the acylation takes place using acetic anhydride as the acylating agent in common protic organic solvents, such as methanol or ethanol, preferably ethanol. The reaction is completed after heating at the boiling point of the selected solvent for about 1 h. The reaction mixture is worked up using known methods in the art. The final product, compound of Formula VI, is isolated in pharmaceutical acceptable purity after recrystallization from commonly used solvents such as a mixture of water and ethanol.

In a further embodiment of the invention, Agomelatine is prepared through an allylic rearrangement from compound of formula la, which is prepared from tetralone of formula VII.

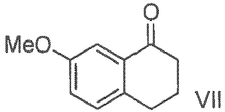

### EXAMPLES

### Example 1: Preparation of compound IIIa, Y=chloro

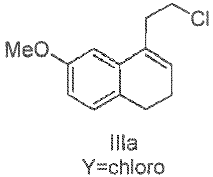

Compound of Formula la, synthesized according to procedure known in the literature (2.51 g), is dissolved in 60 ml THF. Concentrated HCl (37 % w/w, 3.1 ml) is added at room temperature. The reaction mixture is stirred for 1 h and then quenched with aqueous NaOH (10 %w/w, 10 ml), followed by H₂O (40 ml). The reaction mixture is extracted with ethyl acetate (40 ml). Organic phase is separated and the aqueous one is extracted again with ethyl acetate (60 ml). The combined organic phases are dried over anhydrous sodium sulfate, filtered and concentrated. The residue is dissolved in 25 ml CHCl₃ and stirred at room temperature for 24 h. The solvent is removed under vacuum to give 1.92 g of compound of Formula IIIa, Y=chloro.

### Example 2: Preparation of compound IIIa, Y=chloro

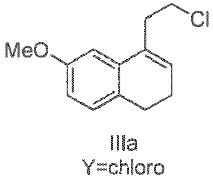

Compound of Formula la, synthesized according to procedure known in the literature (245 g), is dissolved in 2.45 L dichloromethane. Concentrated HCl (37 % w/w, 308 ml) is added at room temperature. The reaction mixture is stirred for 48 h and then cooled down at below 15°C and quenched with aqueous NaOH (10 %w/w, 10 ml). Organic phase is dried over anhydrous sodium sulfate and filtered. The solvent is removed under vacuum to give 257 g of compound of Formula IIIa, Y=chloro.
LC-MS: m/z 223 (100%), 224 (18%), 225 (35%)
HRMS (ESI): calcd for C₁₃H₁₆ClO [M+H]⁺ 223.08897; found 223.08851.
¹H NMR (500 MHz, CDCl₃): δ 7.07 (d, J = 8.2 Hz, 1H), 6.79 (d, J = 2.6 Hz, 1H), 6.70 (dd, J = 8.2, 2.6 Hz, 1H), 5.98 (tt, J = 4.6, 1.0 Hz, 1H), 3.81 (s, 3H), 3.66 (t, J = 7.6 Hz, 2H), 2.90 (td, J = 7.6.2, 1.0 Hz, 2H), 2.68 (t, J = 8.0 Hz, 2H), 2.30 - 2.21 (m, 2H).
¹³C NMR (75MHz, CDCl₃) δ 158.63, 135.05, 132.98, 128.98, 128.39, 128.20, 111.26, 109.44, 55.43, 43.19, 36.34, 27.31, 23.51.

### Example 3: Preparation of compound IVa (Y=chloro)

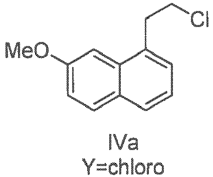

A 150 ml glass sealed tube is charged with a solution of compound IIIa (1.00 g) in toluene. Allyl methacrylate (1.89 g) is added followed by Pd(OH)₂/C (0.22 g, 5 mmol %). The tube is capped tightly and heated at 120 °C for 48 h. Reaction mixture is cooled down to room temperature, filtrated through a celite pad and washed successively with dichloromethane, ethyl acetate and methanol. The filtrate is concentrated to dryness to deliver compound IVa (Y=chloro).

### Example 4: Preparation of compound IVa (Y=chloro)

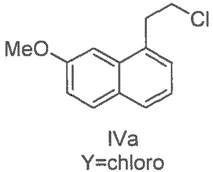

A 5 L stainless steel high pressure reactor is charged with a solution of compound IIIa (257 g) in 3.34 L acetonitrile. Solid DDQ (26.2 g) is added in one portion followed by NaNO₂ (8.0 g). The reactor is capped and a stream of O₂ gas is purged into the solution. Pressure is allowed to build up inside the reactor, which is heated up to 85 - 100°C for 4 h. Reaction mixture is cooled down to room temperature and the solvent is removed under vacuum. The residue in dissolved in toluene (1.93 L) and aqueous Na₂S₂O₅ (10% w/v, 0.76 L) is added. The mixture is stirred for 30 min and filtrated through a celite pad. Organic phase is dried over anhydrous sodium sulfate and filtered. The filtrate is concentrated to dryness to give 251.3g of compound IVa (Y=chloro).
HRMS (ESI): calcd for C₁₃H₁₃ClONa [M+Na]⁺ 243.05526; found: 243. 05474.
¹H NMR (500 MHz, CDCl₃): δ 7.79 (d, J = 8.9 Hz, 1H), 7.72 (d, J = 8.1 Hz, 1H), 7.36 (d, J = 6.9 Hz, 1H), 7.32 - 7.28 (m, 1H), 7.26 (d, J = 2.5 Hz, 1H), 7.19 (dd, J = 9.0, 2.5 Hz, 1H), 3.97 (s, 3H), 3.85 (t, J = 7.9 Hz, 2H), 3.51 (t, J = 7.8 Hz, 2H).
¹³C NMR (126 MHz, CDCl₃) δ 157.9, 132.7, 132.5, 130.4, 129.2, 127.4, 127.3, 123.1, 117.9, 101.9, 55.2, 43.8, 36.5.

### Example 5: Preparation of compound IVa (Y=chloro)

Alternatively, compound of Formula IIIa (1.00 g) is dissolved in toluene and DDQ (1.22 g) is added in one portion at room temperature. The reaction mixture is stirred at the same temperature for 1 h before brine (40 ml) is added. The mixture is extracted with cyclohexane (2 x 40 ml), dried over anhydrous sodium sulfate, filtered and concentrated under vacuum to deliver compound of Formula IVa.

### Example 6: Preparation of compound Va (Hydrochloride salt of V)

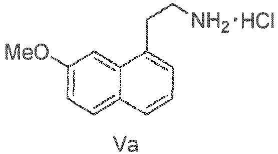

A 150 ml glass sealed tube is charged with a solution of compound IVa (Y=chloro) (0.44 g) in THF (4.0 ml). aq. ammonia (25 % w/w, 6.0 ml) is added and the tube is capped tightly and heated at 110 °C for 12 h. Reaction mixture is cooled down to room temperature, the organic phase is separated and the aqueous one is extracted with ethyl acetate (3 x 20.0 ml). The combined organic phases are dried over anhydrous sodium sulfate, filtered and concentrated to give 0.46 g of compound Va.

### Example 7: Preparation of compound Va (Hydrochloride salt of V)

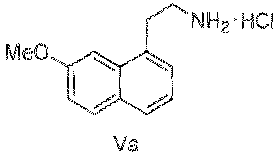

A 5 L stainless steel high pressure reactor is charged with a solution of compound IVa (Y=chloro) (251 g) in ethanol (1.63 L). Aqueous ammonia (25 % w/w, 1.63 L) is added under stirring and the reactor is sealed firmly and heated at 95 - 110 °C for 6 h. Reaction mixture is cooled down to room temperature and the solvents are removed under vacuum. Demineralized water (1.8 L) is added under vigorous stirring followed by addition of 0.63 L dichloromethane. The biphasic system is briefly stirred and the organic phase is separated. The organic phase is washed twice with demineralized water (2 x 0.72 L). The combined aqueous phases are evaporated to afford a brown wet cake. Methanol (0.5 L) is added and evaporated down to dryness. Dichloromethane (0.75 L) is added to the residue and the resulting slurry is stirred vigorously for 1h. The solvent is removed under vacuum and spray wash with dicloromethane to give 166.2 g of compound Va.
HRMS (ESI): calcd for C₁₃H₁₆NO [M-Cl]⁺ 202.12319; found: 202.12285.
¹H NMR (500 MHz, DMSO-*d*₆) δ 8.00 (s, 1H), 7.88 (d, *J* = 8.9 Hz, 1H), 7.78 (d, *J =* 8.0 Hz, 1H), 7.53 (d, *J* = 2.4 Hz, 1H), 7.41 (d, *J* = 6.6 Hz, 1H), 7.32 (d, *J* = 8.0 Hz, 1H), 7.22 (dd, *J* = 8.9, 2.4 Hz, 1H), 3.99 (s, 1H), 3.45 (dd, *J* = 9.7, 6.9 Hz, 1H), 3.09 (dd, *J* = 9.7, 6.9 Hz, 1H).
¹³C NMR (126 MHz, DMSO-*d*₆) δ 157.8, 132.6, 132.4, 130.3, 128.9, 127.5, 127.2, 123.2, 118.2, 102.5, 55.9, 39.4, 30.4.

### Example 8: Preparation of Agomelatine (Formula VI)

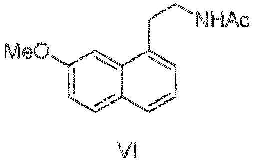

To a solution of compound Va (2.0 g) in ethanol, sodium acetate (2.9 g) and acetic anhydride (2.3 g) are added successively. The resulting mixture is heated to reflux until full consumption of the starting material. Water (20.0 ml) is added and mixture is stirred for 10 min and then extracted with ethyl acetate (2 x 20.0 ml). The combined organic phases are dried over anhydrous sodium sulfate, filtered and concentrated under vacuum to deliver compound of Formula VI (Agomelatine).
HRMS (ESI): calcd for C₁₅H₁₈NO₂ [M+H]⁺ 244.13335; found: 244.13321.
¹H NMR (500 MHz, CDCl₃): δ 7.76 (d, *J* = 8.9 Hz, 1H), 7.69 (d, *J* = 7.0 Hz, 1H), 7.47 (d, *J* = 2.4 Hz, 1H), 7.31-7.24 (m, 2H), 7.19 (dd, *J* = 8.9, 2.4 Hz, 1H), 5.63 (brs, 1H), 3.99 (s, 3H), 3.62 (q, *J* = 7.0 Hz, 2H), 3.25 (t, *J* = 7.0 Hz, 2H), 1.96 (s, 3H).
¹³C NMR (126 MHz, CDCl₃) δ 170.3, 157.9, 133.6, 133.2, 130.2, 129.3, 127.1, 127.0, 123.1, 118.3, 102.4, 55.5, 40.1, 33.2, 23.3.

### Overall synthetic route to compound VI

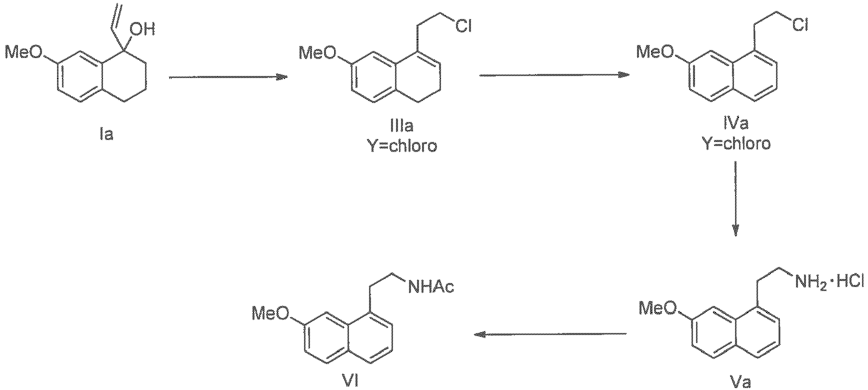

## Claims

1. A process for the preparation of compounds of formula IVa 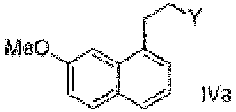 in which
Y is selected from hydroxyl or protected hydroxyl group, halogen, amino or alkylamino group, amido or substituted amido group, or phthalimide group, wherein:
protected hydroxyl means a hydroxyl group suitably protected with a protecting group;
alkylamino means an amino group substituted with one or two of the same or different alkyl groups;
amido means -NHCOX wherein X is selected from an alkyl, alkoxy, alkoxyalkyl or alkylamino group;
substituted amido means -N(Z)COX, wherein X is defined as above and Z is alkyl;
comprising the following steps:
a) allylic rearrangement of a compound of formula la, 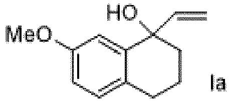 to form compound of formula IIa 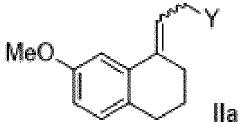 which may be optionally isolated;
b) isomerization of a compound of formula IIa to form a compound of formula IIIa 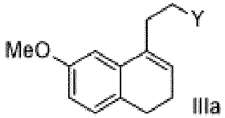 wherein Y is defined as above, which may be optionally isolated;
c) conversion of a compound of formula IIIa to a compound of formula IVa, which may be optionally isolated as a free compound or a salt 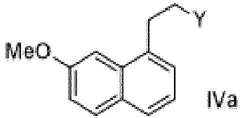 wherein Y is defined as above.

2. A process for the preparation of compounds of formula IVa as claimed in claim 1, wherein an addition step is performed, wherein Y is converted to an amido group, preferably NHCOCH₃, when Y is other than an amido group.

3. A process according to claims 1 or 2, wherein step c is performed with the use of a catalytic system comprising 2,3-Dichloro-5,6-dicyano-1,4-benzoquinone (DDQ).

4. A process according to claim 3, wherein the catalytic system further comprises co-oxidants selected from sodium nitrite (NaNO₂)/O₂, PbO₂, HClO₄/H₂SO₄, HIO₄, MnO₂, Mn(OAc)3, tert-butylnitrite (TBN)/02, FeCl₃, Pd(OAc)3, HNO₃.

5. A process, according to claims 1 and 2 where Y is halogen.

6. Process, according to claims 1 or 2 further comprising converting a compound of Formula IVa, wherein Y is not amino or amido group,
to a compound of formula V 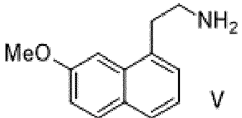 which is then reacted with a suitable acylating agent to produce Agomelatine of formula VI.

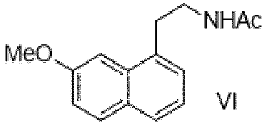

7. Compound of formula IIIa.

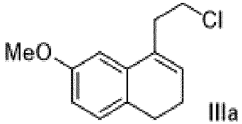

8. Use of compound of formula IIIa, for the preparation of compounds of Formula IVa.

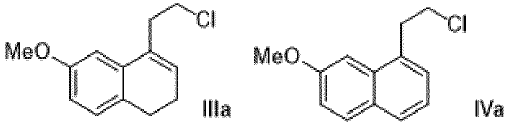

9. Use of compound of formula IIIa for the preparation of Agomelatine of Formula VI.

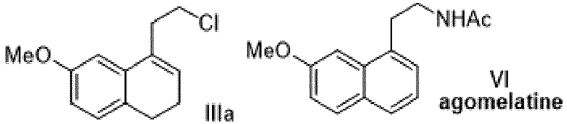

10. Use of compound of formula la, for the preparation of Agomelatine of Formula VI through allylic rearrangement of compound of formula Ia.

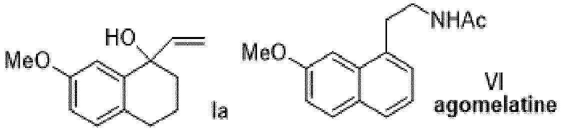

11. Use of tetralone of formula VII for the preparation of Agomelatine through allylic rearrangement of compound of formula Ia.

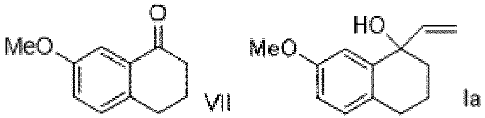

## Patentansprüche

1. Ein Verfahren zur Herstellung von Verbindungen der Formel IVa 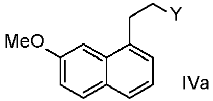 in dem
Y ist ausgewählt aus einer Hydroxyl- oder geschützten Hydroxylgruppe, einer Halogen-, Amino- oder Alkylaminogruppe, einer Amido- oder substituierten Amidogruppe oder einer Phthalimidgruppe, wobei:
geschützte Hydroxylgruppe eine Hydroxylgruppe bedeutet, die geeignet mit einer Schutzgruppe geschützt ist;
Alkylamino bedeutet eine Aminogruppe, die mit einer oder zwei der gleichen oder verschiedenen Alkylgruppen substituiert ist;
Amido bedeutet -NHCOX, wobei X ausgewählt ist aus einer Alkyl-, Alkoxy-, Alkoxyalkyl- oder Alkylaminogruppe;
substituiertes Amido bedeutet -N(Z) COX, wobei X wie oben definiert ist und Z Alkyl ist;
umfassend die folgenden Schritte:
a) allylische Umlagerung einer Verbindung der Formel Ia, 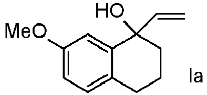 um eine Verbindung der Formel IIa zu bilden 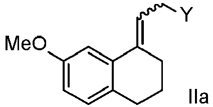 die optional isoliert werden kann;
b) Isomerisierung einer Verbindung der Formel IIa unter Bildung einer Verbindung der Formel IIIa 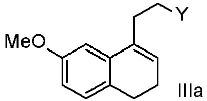 worin Y wie oben definiert ist, das gegebenenfalls isoliert werden kann;
c) Umwandlung einer Verbindung der Formel IIIa in eine Verbindung der Formel IVa, die gegebenenfalls als freie Verbindung oder Salz isoliert werden kann 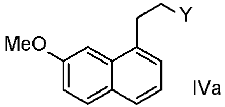 worin Y wie oben definiert ist.

2. Verfahren zur Herstellung von Verbindungen der Formel IVa nach Anspruch 1, wobei ein Additionsschritt durchgeführt wird, wobei Y in eine Amidogruppe, vorzugsweise NHCOCH₃, umgewandelt wird, wenn Y nicht eine Amidogruppe ist.

3. Verfahren nach Anspruch 1 oder 2, wobei Schritt c unter Verwendung eines katalytischen Systems durchgeführt wird, das 2,3-Dichlor-5,6-dicyano-1,4-benzochinon (DDQ) umfasst.

4. Verfahren nach Anspruch 3, wobei das katalytische System ferner Cooxidationsmittel umfasst, ausgewählt aus Natriumnitrit (NaNO₂)/O₂, PbO₂, HClO₄/H₂SO₄, HIO₄, MnO₂, Mn(OAc)₃, tert-Butylnitrit (TBN)/O₂, FeCl₃, Pd(OAc)₃, HNO₃.

5. Verfahren nach Anspruch 1 und 2, bei dem Y Halogen ist.

6. Verfahren nach den Ansprüchen 1 oder 2, ferner umfassend das Umwandeln einer Verbindung der Formel IVa, wobei Y keine Amino- oder Amidogruppe ist,
zu einer Verbindung der Formel V 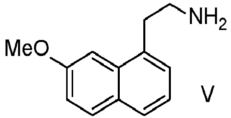 die dann mit einem geeigneten Acylierungsmittel umgesetzt wird, um Agomelatine der Formel VI herzustellen.

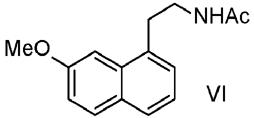

7. Verbindung der Formel IIIa.

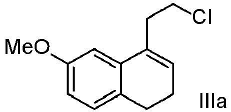

8. Verwendung der Verbindung der Formel IIIa zur Herstellung von Verbindungen der Formel IVa.

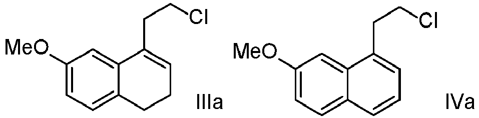

9. Verwendung der Verbindung der Formel IIIa zur Herstellung von Agomelatine der Formel VI.

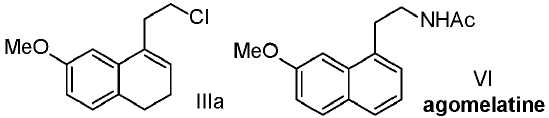

10. Verwendung der Verbindung der Formel Ia zur Herstellung von Agomelatine der Formel VI durch allylische Umlagerung der Verbindung der Formel Ia.

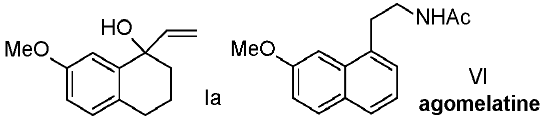

11. Verwendung von Tetralon der Formel VII zur Herstellung von Agomelatine durch allylische Umlagerung der Verbindung der Formel Ia.

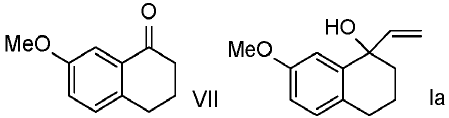

## Revendications

1. Un procédé pour la préparation de composés de formule IVa 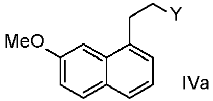 dans laquelle
Y est sélectionné parmi un groupe hydroxyle ou hydroxyle protégé, halogène, un groupe amino ou aminoalkylé, un groupe amido ou amido substitué, ou un groupe phtalamide, dans lequel :
hydroxyle protégé signifie un groupe hydroxyle convenablement protégé par un groupement protecteur ;
alkylamino signifie un groupe amino substitué par 1 ou 2 groupements alkyle identiques ou différents ;
amido signifie -NHCOX où X est sélectionné parmi un alkyle, alkoxy, alkoxyalkyle ou alkylamino groupe ;
amido substitué signifie -N(Z)COX, où X est défini comme ci-dessus et Z est un alkyle ;
comprenant les étapes suivantes :
a) réarrangement allylique du composé de formule la, 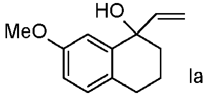 pour former le composé de formule IIa, 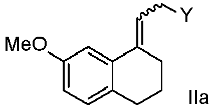 Où Y est tel que défini ci-dessus, pouvant être éventuellement isolé ;
b) isomérisation du composé de formule IIa pour former un composé de formule IIIa, 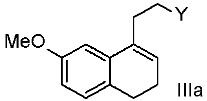 où Y est tel que défini ci-dessus, pouvant être éventuellement isolé ;
c) conversion du composé de formule IIIa en un composé de formule IVa, pouvant être éventuellement isolé sous forme de composé libre ou d'un sel, 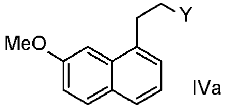 Y étant tel que défini ci-dessus.

2. Un procédé pour la préparation de composés de formule IVa selon la revendication 1, dans lequel une étape supplémentaire est effectuée, dans laquelle Y est converti en un groupe amido, de préférence NHCOCH₃, lorsque Y est autre qu'un groupe amido.

3. Un procédé selon les revendications 1 ou 2, dans lequel l'étape c est effectuée avec l'utilisation d'un système catalytique comprenant la 2,3-dichloro-5,6-dicyano-1,4-benzoquinone (DDQ).

4. Un procédé selon la revendication 3, dans lequel le système catalytique comprend en outre des co-oxidants sélectionnés parmi le nitrite de sodium, (NaNO₂)/O₂, PbO₂, HClO₄/H₂SO₄, HIO₄, MnO₂, Mn(OAc)₃, le tert-butylnitrite, (TBN)/O₂, FeCl₃, Pd(OAc)₃, HNO₃.

5. Un procédé selon les revendications 1 et 2 dans lequel Y est un halogène.

6. Un procédé selon les revendications 1 ou 2, comprenant en outre la conversion du composé de formule IVa, dans laquelle Y n'est pas un groupe amino ou amido, en un composé de formule V, 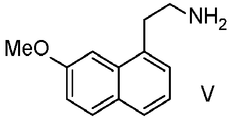 qui est par la suite mis à réagir avec un agent d'acylation approprié pour produire l'Agomélatine de formule VI.

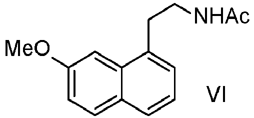

7. Composé de formule IIIa.

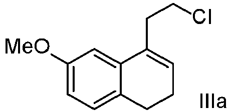

8. Utilisation du composé de formule IIIa pour la préparation de composé de formule IVa.

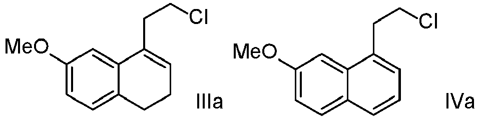

9. Utilisation du composé de formule IIIa pour la préparation de l'Agomélatine de formule VI.

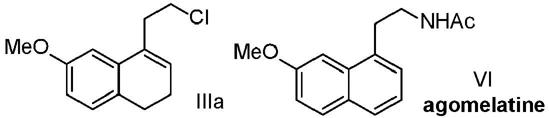

10. Utilisation du composé de formule la pour la préparation de l'Agomélatine de formule VI par réarrangement allylique du composé de formule la.

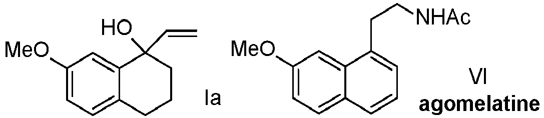

11. Utilisation de tétralone de formule VII pour la préparation de l'Agomélatine par réarrangement allylique du composé de formule la.
